# EUROPEAN PATENT SPECIFICATION

(11) **EP 3 658 106 B1**
(45) Date of publication and mention of the grant of the patent: **24.09.2025**
(21) Application number: 18721562.9
(22) Date of filing: 10.04.2018
(51) Int. Cl.: A61K 8/27, A61K 8/21, A61K 8/19, A61K 8/73, A61K 8/92, A61K 8/9789, A61K 8/34, A61K 8/35, A61K 8/98, A61Q 11/00

(54) **ORAL CARE COMPOSITIONS**
MUNDPFLEGEZUSAMMENSETZUNGEN
COMPOSITIONS POUR SOINS D'HYGIÈNE BUCCO-DENTAIRE

(30) Priority: 31.08.2017 IN 201741030944
(43) Date of publication of application: 03.06.2020
(73) Proprietor: Colgate-Palmolive Company, New York, NY 10022 (US)
(72) Inventor: JHA, Manisha, Houston 77007,TX (US); UTGIKAR, Neelima, Dombivli (E) Maharashtra 421203 (IN); POTNIS, Shashank, Princeton 08540, NJ (US); PLATA, Rolando, Las Pinas City 1751 (PH); SREENIVASAN, Prem, Westfield, New Jersey 07090 (US); SUN, Fusong, Hillsborough 08844,NJ (US)
(74) Representative: Wibbelmann, Jobst
(86) International application number: PCT/US2018/026885
(87) International publication number: WO 2019/045781

(56) References cited:
- WO-A1-2014/203263
- WO-A1-2015/095627
- US-A1- 2004 219 183
- US-A1- 2007 116 652

## Description

There is an interest in providing oral care products which have a natural flavor profile. In particular, oral care products containing a mixture of herbal ingredients, such as those reported in Ayurvedic texts.

However, the degree of antibacterial action achieved with herbal ingredients is not as complete as would be desired. It would therefore be desirable to provide such oral care products containing herbal ingredients having enhanced antibacterial effectiveness.

Embodiments of the present invention provide compositions which address, *inter alia,* this problem.

### BRIEF SUMMARY

The present invention provides an oral care composition comprising a first source of zinc ions comprising zinc oxide; a second source of zinc ions comprising zinc citrate; a gum system comprising carrageenan; an orally acceptable carrier comprising calcium carbonate; and a natural ingredient component comprising: an herbal extract; and an essential oil. The invention also refers to said compositions for use in methods as defined in the claims.

The present invention also provides a composition for use in a method of treating or preventing a disease or condition of the oral cavity, the treating or preventing comprising contacting an oral cavity surface of a patient in need thereof with the composition, wherein the composition is any one of the compositions described herein. WO 2015/095627 A1 discloses an oral care composition comprising zinc oxide and zinc citrate wherein the weight ratio of zinc oxide:zinc citrate is 1.5: 1 to 4.5: 1, wherein the composition can contain herbal extracts. WO 2014/203263 A1 discloses an antibacterial combination of essential oils comprising (i) bisabolol and one or more essential oils selected from citral, carvacrol, oregano extract, and rosemary, or (ii) carvacrol and one or more essential oils selected from bisabolol, thymol, and rosemary.

### DETAILED DESCRIPTION

It has been surprisingly found that the current formulations offer the advantage of providing a Naturals flavor profile along with a robust antimicrobial protection, without significantly interfering with the stability of the oral care composition and by allowing for formulations which avoid undesirable aesthetic qualities (e.g., poor taste).

The compositions described have a surprisingly advantageous combination of good viscosity and stability of the natural ingredients which was not shared by formulations of similar composition, if they did not include all of the first source of zinc comprising zinc oxide; a second source of zinc comprising zinc citrate; a gum system comprising carrageenan; a calcium source comprising natural calcium carbonate and precipitated calcium carbonate; and herbal extract and essential oil.

The invention is as defined in the claims and contemplates any of the following aspects (unless otherwise indicated, values are given as percentage of the overall weight of the composition).

The ratio of the amount of zinc oxide (e.g., wt.%) to zinc citrate (e.g., wt%) can be from 1.5:1 to 4.5:1 (e.g., 2:1, 2.5:1, 3:1, 3.5:1, or 4:1). The zinc citrate can be present in an amount of from 0.25 to 1 wt% (e.g., 0.5 wt. %) and zinc oxide may be present in an amount of from 0.75 to 1.25 wt% (e.g., 1.0 wt. %) based on the weight of the oral care composition. The zinc citrate can be present in an amount of from 0.3 to 0.8 wt% (e.g., 0.5 wt. %) and zinc oxide may be present in an amount of from 0.8 to 1.2 wt% (e.g., 1.0 wt. %) based on the weight of the oral care composition. The zinc citrate can be present in an amount of from 0.4 to 0.6 wt% (e.g., 0.5 wt. %) and zinc oxide may be present in an amount of from 0.9 to 1.1 wt% (e.g., 1.0 wt. %) based on the weight of the oral care composition. The zinc citrate can be about 0.5 wt% (e.g., zinc citrate trihydrate). The zinc oxide can be about 1.0 wt%. The zinc citrate can be about 0.5 wt% and the zinc oxide can be about 1.0 wt%. The composition contains one or more herbal extract(s) and one or more essential oils which can be selected from the group consisting of amla extract, honey extract, almond extract, aloe vera extract, maricha extract, ginger extract, fenugreek, neem seed oil, sesame oil, cinnamon leaf oil, clove oil, thyme oil, eucalyptus oil, eugenol, menthol, babool and camphor. The herbal extracts and essential oils can be selected from amla extract, honey extract, neem seed oil, basil oil, aloe vera extract, cinnamon leaf oil, clove oil, and camphor. The carrageenan gum system can comprise carrageenan concentrate.

As used throughout, ranges are used as a short hand for describing each and every value that is within the range. Any value within the range can be selected as the terminus of the range.

In some embodiments, the compositions further comprise one or more components selected from a fluoride ion source; a tartar control agent; a buffering agent; an antibacterial agent; an abrasive; and a combination of two or more thereof.

Some embodiments provide compositions wherein at least one of the one or more components is a fluoride ion source selected from: stannous fluoride, sodium fluoride, potassium fluoride, sodium monofluorophosphate, sodium fluorosilicate, ammonium fluorosilicate, amine fluoride, ammonium fluoride, and a combination of two or more thereof.

Other optional additives may be included. Among such optional additives, included are those provided in order to change appearance or aesthetic appeal, and/or to preserve the final product, and/or for taste/cosmetic appeal and/or as therapeutic and prophylactic ingredients for oral health, prevention or treatment of a condition or disorder of hard or soft tissue of the oral cavity, or the prevention or treatment of a physiological disorder or condition.

Some embodiments provide a composition wherein a preservative is present. In some embodiments, the preservative is selected from parabens, potassium sorbate, benzyl alcohol, phenoxyethanol, polyaminopropryl biguanide, caprylic acid, sodium benzoate and cetylpyridinium chloride. In some embodiments, the preservative is present at a concentration of about 0.0001 to about 1%, by weight.

Colorants such as dyes may be food color additives presently certified under the Food Drug & Cosmetic Act for use in food and ingested drugs, including dyes such as FD&C Red No. 3 (sodium salt of tetraiodofluorescein), Food Red 17, disodium salt of 6-hydroxy-5-{(2-methoxy-5-methyl-4-sulphophenyl)azo}-2-n- aphthalenesulfonic acid, Food Yellow 13, sodium salt of a mixture of the mono and disulphonic acids of quinophtalone or 2-(2-quinolyl) indanedione, FD&C Yellow No. 5 (sodium salt of 4-p-sulfophenylazo-1-p-sulfophenyl-5-hydroxypyrazole-3 carboxylic acid), FD&C Yellow No. 6 (sodium salt of p-sulfophenylazo-B-naphtol-6-monosulfonate), FD&C Green No. 3 (disodium salt of 4-{[4-(N-ethyl-p-sulfobenzylamino)-phenyl]-(4-hydroxy-2-sulfoniumphenyl)-methylene}-[1-(N-ethyl-N-p-sulfobenzyl)-DELTA-3,5-cycl-ohexadienimine], FD&C Blue No. 1 (disodium salt of dibenzyldiethyl-diamino- triphenylcarbinol trisulfonic acid anhydrite), FD&C Blue No. 2 (sodium salt of disulfonic acid of indigotin) and mixtures thereof in various proportions. Typically, colorants if included are present in very small quantities.

Flavor profile is provided by various ingredients which include, but are not limited to, herbal extracts and essential oils (e.g., rosemary extract, tea extract, magnolia extract, thymol, menthol, eucalyptol, geraniol, carvacrol, citral, hinokitol, catechol, methyl salicylate, epigallocatechin gallate, epigallocatechin, gallic acid, miswak extract, sea-buckthorn extract) synthetic flavor oils and flavoring aromatics, and/or oils, oleo resins and extracts derived from herbs plants, leaves, flowers, fruits, honey, and combinations thereof. Representative herbal extracts include: spearmint oil, cinnamon oil, peppermint oil, clove oil, bay oil, thyme oil, cedar leaf oil, oil of nutmeg, oil of sage, and oil of bitter almonds. Examples of herbal extracts include mints such as peppermint, artificial vanilla, cinnamon derivatives, and various fruit flavors, whether employed individually or in admixture. Generally, any flavoring agent or food additive, such as those described in Chemicals Used in Food Processing, publication 1274 by the National Academy of Sciences, pages 63-258, may be used. Typically, the total amount of herbal extract or essential oil is from about 0.1 to about 5%, by weight; from about 0.2 to about 4% by weight; about 0.5 to about 3% by weight; about 1 to about 2.5% by weight; from 1 to about 3% by weight.

A preferred group of herbal extracts and essential oils include amla extract, honey extract, almond extract, aloe vera extract, maricha extract, ginger extract, fenugreek, neem seed oil, sesame oil, cinnamon leaf oil, clove oil, thyme oil, eucalyptus oil, eugenol, menthol, babool and camphor.

Sweeteners include both natural and artificial sweeteners. Suitable sweeteners include water soluble sweetening agents such as monosaccharides, disaccharides and poysaccharides such as xylose, ribose, glucose (dextrose), mannose, galactose, fructose (levulose), sucrose (sugar), maltose, water soluble artificial sweeteners such as the soluble saccharin salts, i.e., sodium or calcium saccharin salts, cyclamate salts dipeptide based sweeteners, such a L-aspartic acid derived sweeteners, such as L-aspartyl-L-phenylalaine methyl ester (aspartame). In general, the effective amount of sweetener is utilized to provide the level of sweetness desired for a particular composition, will vary with the sweetener selected. This amount will normally be from about 0.001 to about 5%, by weight. In some embodiments, the sweetener is sodium saccharin and is present at a concentration of about 0.01%, by weight.

Whitening agents, material which is effective to effect whitening of a tooth surface to which it is applied, such as hydrogen peroxide and urea peroxide, high cleaning silica, preservatives, silicones, and chlorophyll compounds may be incorporated into the compositions of the present invention. In various embodiments, the compositions of this invention comprise a peroxide whitening agent, comprising a peroxide compound. A peroxide compound is an oxidizing compound comprising a bivalent oxygen-oxygen group. Peroxide compounds include peroxides and hydroperoxides, such as hydrogen peroxide, peroxides of alkali and alkaline earth metals, organic peroxy compounds, peroxy acids, pharmaceutically-acceptable salts thereof, and mixtures thereof. Peroxides of alkali and alkaline earth metals include lithium peroxide, potassium peroxide, sodium peroxide, magnesium peroxide, calcium peroxide, barium peroxide, and mixtures thereof. Organic peroxy compounds include carbamide peroxide (also known as urea hydrogen peroxide), glyceryl hydrogen peroxide, alkyl hydrogen peroxides, dialkyl peroxides, alkyl peroxy acids, peroxy esters, diacyl peroxides, benzoyl peroxide, and monoperoxyphthalate, and mixtures thereof. Peroxy acids and their salts include organic peroxy acids such as alkyl peroxy acids, and monoperoxyphthalate and mixtures thereof, as well as inorganic peroxy acid salts such as persulfate, dipersulfate, percarbonate, perphosphate, perborate and persilicate salts of alkali and alkaline earth metals such as lithium, potassium, sodium, magnesium, calcium and barium, and mixtures thereof. In various embodiments, the peroxide compound comprises hydrogen peroxide, urea peroxide, sodium percarbonate and mixtures thereof. In some embodiments, the peroxide compound comprises hydrogen peroxide. In some embodiments, the peroxide compound consists essentially of hydrogen peroxide. In some embodiments a non-peroxide whitening agent may be provided. Whitening agents among those useful herein include non-peroxy compounds, such as chlorine dioxide, chlorites and hypochlorites. Chlorites and hypochlorites include those of alkali and alkaline earth metals such as lithium, potassium, sodium, magnesium, calcium and barium. Non-peroxide whitening agents also include colorants, such as titanium dioxide and hydroxyapatite. One or more whitening agents are optionally present in a tooth-whitening effective total amount. In some embodiments the whitening agent is separated from the aqueous carrier. In some embodiments the whitening agent is separated from the aqueous carrier by encapsulation of the whitening agent.

Optionally, breath freshening agents may be provided. Any orally acceptable breath freshening agent can be used, including without limitation. One or more breath freshening agents are optionally present in a breath freshening effective total amount.

Other embodiments provide compositions wherein at least one of the one or more components is a tartar control agent. Tartar control agents among those useful herein include phosphates and polyphosphates (for example pyrophosphates), polyaminopropanesulfonic acid (AMPS), polyolefin sulfonates, polyolefin phosphates, diphosphonates such as azacycloalkane-2,2-diphosphonates (e.g., azacycloheptane-2,2-diphosphonic acid), N-methyl azacyclopentane-2,3-diphosphonic acid, ethane-1-hydroxy-1,1-diphosphonic acid (EHDP) and ethane-1-amino-1,1-diphosphonate, phosphonoalkane carboxylic acids and salts of any of these agents, for example their alkali metal and ammonium salts. Useful inorganic phosphate and polyphosphate salts include monobasic, dibasic and tribasic sodium phosphates, sodium tripolyphosphate, tetrapolyphosphate, mono-, di-, tri- and tetrasodium pyrophosphates, sodium trimetaphosphate, sodium hexametaphosphate and mixtures thereof, wherein sodium can optionally be replaced by potassium or ammonium. Other useful anticalculus agents include polycarboxylate polymers and polyvinyl methyl ether/maleic anhydride (PVME/MA) copolymers, such as those available under the Gantrez^{™} brand from ISP, Wayne, N.J. In some embodiments, a phosphate is present at a concentration of from about 0.01 to about 10%, by weight. In some embodiments, a phosphate is present at a concentration of from about 1%, by weight.

Some embodiments provide compositions wherein a buffering agent is present. In some embodiments, sodium phosphate monobasic is present at a concentration of from about 0.01 to about 5%, by weight. In some embodiments, sodium phosphate monobasic is present at a concentration of about 1%, by weight. In some embodiments, sodium phosphate dibasic is present at a concentration of from about 0.01 to about 5%, by weight. In some embodiments, sodium phosphate dibasic is present at a concentration of about 0.15%, by weight.

Antioxidants are another class of optional additives. Any orally acceptable antioxidant can be used, including butylated hydroxyanisole (BHA), butylated hydroxytoluene (BHT), vitamin A, carotenoids, vitamin E, flavonoids, polyphenols, ascorbic acid, herbal antioxidants, chlorophyll, melatonin, and mixtures thereof.

Also optional, a saliva stimulating agent, useful for example in amelioration of dry mouth, may be included. Any orally acceptable saliva stimulating agent can be used, including without limitation food acids such as citric, lactic, malic, succinic, ascorbic, adipic, fumaric, and tartaric acids, and mixtures thereof. One or more saliva stimulating agents are optionally present in a saliva stimulating effective total amount.

Optionally, an antiplaque (e.g., plaque disrupting) agent may be included. Any orally acceptable antiplaque agent can be used, including without limitation stannous, copper, magnesium and strontium salts, dimethicone copolyols such as cetyl dimethicone copolyol, papain, glucoamylase, glucose oxidase, urea, calcium lactate, calcium glycerophosphate, strontium polyacrylates and mixtures thereof.

Optional desensitizing agents include potassium citrate, potassium chloride, potassium tartrate, potassium bicarbonate, potassium oxalate, potassium nitrate, strontium salts, and mixtures thereof.

Optional additives also include vitamins, and proteins. Vitamins include Vitamins C and D, thiamine, riboflavin, calcium pantothenate, niacin, folic acid, nicotinamide, pyridoxine, cyanocobalamin, para-aminobenzoic acid, bioflavonoids, pantheon, retinyl palmitate, tocopherol acetate, and mixtures thereof. Suitable proteins include milk proteins and enzymes such as peroxide-producing enzymes, amylase, plaque-disrupting agents such as papain, glucoamylase, glucose oxidase, and "next generation" enzymes."

In some embodiments, the composition has a free water content of greater than about 10%, by weight. In some embodiments, the composition has a free water content of greater than about 11%, by weight. In other embodiments, the composition has a free water content of greater than about 12%, by weight. Yet other embodiments provide compositions wherein the free water content is greater than about 13%, by weight. Still other embodiments provide compositions having a free water content of greater than about 14%, by weight. In some embodiments, the composition has a free water content of greater than about 15%, by weight. While other embodiments provide compositions have a free water content of greater than about 16%, by weight. In some embodiments, the composition has a free water content of about 17%, by weight. In some embodiments, the composition has a free water content of greater than about 17%, by weight. In some embodiments, the composition has a free water content of from about 10% to about 20%, by weight.

Some embodiments provide a composition for use in a method of treating or preventing a disease or condition of the oral cavity, the treating or preventing comprising contacting an oral cavity surface of a patient in need thereof with the composition, wherein the composition is any one of the compositions described herein. The disease or condition of the oral cavity can be halitosis. In some embodiments, the present invention provides a composition for reducing volatile sulfur compounds in the oral cavity of a subject in need thereof. In further embodiments, the present invention provides a composition for increasing the delivery of a metal ion to an oral cavity surface.

The orally acceptable carrier comprises calcium carbonate but the specific composition of the carrier preferably depends on the intended use of the composition. In various embodiments, the carrier is aqueous, comprising from about 5 to about 95%, by weight, water or from about 10 to about 70%, by weight, water. In other embodiments, the carrier is substantially non-aqueous. In a dentifrice carrier, water content can be from about 5 to about 70%, from about 10 to about 50%, or from about 20 to about 40%, by weight.

The carrier is as defined in the claims but may further comprise any of a variety of materials, including emulsifiers, thickeners, fillers, and preservatives. In some embodiments, the carrier may include a functional or active material, such as those described above.

In some embodiments, the carrier comprises a humectant, such as glycerin, sorbitol or an alkylene glycol such as polyethylene glycol or propylene glycol. In some embodiments, the carrier comprises a humectant at a level of from about 10 to about 80% by weight, or about 20 to about 60% by weight of the composition. Carrier compositions among those useful herein are disclosed in U.S. Patents 5,695,746 to Garlick, Jr., et al. and 4,839,157 to Mei-King Ng et al.

The composition comprises a gum system comprising carrageenan. Thickeners or gelling agents useful herein include inorganic, natural or synthetic thickeners or gelling agents. In some configurations, the carrier comprises the thickener and gelling agent at total levels of from about 0.1 to about 15% by weight, or from about 0.4 to about 10% by weight of the composition. Examples of thickeners and gelling agents useful herein include inorganic thickening silicas such as: an amorphous silica, for example Zeodent^{®} 165 (Huber Corporation); Irish moss; iota-carrageenan; gum tragacanth; or polyvinylpyrrolidone. The preferred thickener is carrageenan concentrate. In some embodiments, the amount of carrageenan concentrate is from about 0.5% to about 2% by weight; from about 0.5 to about 1.5% by weight, about 0.7 to about 1.2% by weight.

In certain embodiments, the carrier comprises an abrasive or polishing agent comprising a combination of refined natural calcium carbonate and precipitated calcium carbonate. Optional abrasive include a calcined alumina, sodium bicarbonate, dicalcium phosphate or calcium pyrophosphate. In various embodiments, the carrier is clear. In various embodiments, the carrier comprises an abrasive at a level of from about 5 to about 70% by weight of the composition; from about 20 to about 60 % by weight; from about 30 to about 50 percent by weight; from about 35 to about 45 % by weight.

In some embodiments, the compositions comprise a surfactant or mixture of surfactants. Surfactants among those useful herein include water-soluble salts of at least one higher fatty acid monoglyceride monosulfate, such as the sodium salt of the monsulfated monoglyceride of hydrogenated coconut oil fatty acids; cocamidopropyl betaine; a higher alkyl sulfate such as sodium lauryl sulfate; an alkyl aryl sulfonate such as sodium dodecyl benzene sulfonate; a higher alkyl sulfoacetate; sodium lauryl sulfoacetate; a higher fatty acid ester of 1,2-dihydroxy propane sulfonate; and a substantially saturated higher aliphatic acyl amides of a lower aliphatic amino carboxylic acid, such as those having 12 to 16 carbons in the fatty acid, alkyl or acyl radicals; and mixtures thereof. Amides can be, for example, N-lauroyl sarcosine, and the sodium, potassium, and ethanolamine salts of N-lauroyl, N-myristoyl, or N-palmitoyl sarcosine. In various embodiments, the surfactant is present at a concentration of from about 0.3 to about 5% by weight of composition, or about 0.5 to about 3% by weight of composition.

Compositions as described herein can be prepared according to methods readily known to those skilled in the art.

Embodiments of the present invention are further described in the following examples. The examples are merely illustrative and do not in any way limit the scope of the invention as described and claimed.

### EXAMPLES

A series of formulations is prepared and evaluated on a range of physical characteristics, and display unacceptable performance either as initially formulated or upon storage under the stated conditions, as detailed below.

### Comparative Example A

A composition with a chalk (calcium carbonate) backbone is prepared for use in Comparative Example A as described in Table 1 (below).

**Table 1**

| **Ingredient** | **Wt.%** |
|---|---|
| Humectant (70%) Solution | 18.00 |
| Abrasive | 1.00 |
| Purified water | 26.38 |
| CarbobxyMethyl Cellulose TMS | 1.00 |
| Sweetener | 0.22 |
| Refined Natural Calcium Carbonate | 35.00 |
| Precipitated Calcium Carbonate | 9.00 |
| Abrasive | 0.75 |
| Sodium Monofluorophosphate-USP | 0.76 |
| pH Adjusting Agent | 0.50 |
| Anionic Surfactant | 2.00 |
| Zinc Oxide | 1.00 |
| Zinc Citrate | 0.50 |
| Potassium Nitrate | 0.50 |
| Benzyl Alcohol | 0.30 |
| Flavor (containing essential oils) | 1.80 |
| Amla Extract | 1.00 |
| Honey Extract | 0.0004 |
| Almond Extract | 0.0004 |
| Neem Seed Oil | 0.010 |
| Aloe Vera Extract | 0.0004 |
| Sesame Oil | 0.0004 |
| Cinnamon leaf oil | 0.0004 |
| Colorants | 0.28 |
| TOTAL | 100.0 |

Physical properties of the compositions are tested upon completion of formulating at 25°C, and after storage for the time period indicated, under the stated conditions of temperature and relative humidity (R.H.). Upon initial testing, the Brookfield viscosity of these slurry composition was measured and the values reported are expressed as bku, which is a machine based measurement of relative resistance to deformation, used here as a shorthand comparator within these slurry systems. On the bku scale, the desired viscosity for toothpaste in the Naturals segment is around 25-30 bku. The viscosity of this sample is observed to be undesirably high (above 50 bku). The product with this formula is therefore difficult to squeeze from a tube. In addition, discoloration in the formula is observed, and separation of herbal extracts is observed. Upon storage, the product is consistently hard to squeeze due to high viscosity, and separation of the composition was observed, as described in Table 2 (below).

**Table 2**

| **Conditions** | **Time** | **Viscosity (bku)** | **Physical Examination of Dental Cream** |
|---|---|---|---|
| 25°C | 0 mo | 50.4 | PASS, Acceptable |
| 25°C/60%RH | 1 mo | 48 | PASS, Acceptable |
| 25°C/60%RH | 2 mo | 52.7 | Not Acceptable Appearance, Separation |
| 25°C/60%RH | 3 mo | 50.0 | Not Acceptable, Appearance, Hard to squeeze, Amla separation, Discoloration due to Amla separation |
| 40°C/75%RH | 1 mo | 47 | PASS, Acceptable |
| 40°C/75%RH | 2 mo | 49.8 | Not Acceptable Appearance, Separation |
| 40°C/75%RH | 3 mo | 48.3 | Not Acceptable Appearance, Hard to squeeze, Amla separation, Discoloration due to Amla separation |
| 49°C | 4 wk | - | PASS, Acceptable |
| 4°C | 24 hr | - | PASS, Acceptable |

### Comparative Example B

A composition with silica backbone is prepared for use in Comparative Example B and formulated as described in Table 3 (below).

**Table 3**

| **Ingredient** | **Wt. %** |
|---|---|
| Humectant (70%) | 68.00 |
| PEG 600 | 1.00 |
| Carboxymethyl Cellulose | 0.50 |
| Sweetener | 0.15 |
| Purified Water | 6.15 |
| Sodium Fluoride | 0.22 |
| Maricha (Piper nigrum) | 0.70 |
| Shunthi / Dry Ginger extract (Zingiber officinale) | 0.70 |
| Clove Oil | 0.30 |
| Thymol | 0.20 |
| Eucalyptus Oil | 0.030 |
| Eugenol | 0.20 |
| Babool | 0.50 |
| Fenugreek | 0.10 |
| Karpura / Camphor (Cinnamomum Camphora) | 0.40 |
| Zinc Oxide | 1.00 |
| Zinc Citrate | 0.50 |
| Amla Extract | 0.20 |
| Colorants | 0.35 |
| Abrasive | 8.0 |
| Thickener | 8.0 |
| Anionic Surfactant | 2.0 |
| Menthol | 0.50 |
| Benzyl Alcohol | 0.30 |
| TOTAL | 100.0 |

The composition is evaluated and the results are reported in Table 4 (below).

**Table 4**

| **Condition** | **Time** | **Viscosity (bku)** | **Physical Examination of Dental Cream** |
|---|---|---|---|
| 25°C | 0 mo | 9 | Acceptable for physical parameters |
| 25°C/60%RH | 1 mo | 12 | Low on stand-up, Acceptable for physical parameters |
| 25°C/60%RH | 2 mo | 21.24 | Acceptable |
| 25°C/60%RH | 3 mo | 30.39 | Hard to Squeeze, Acceptable |
| 25°C/60%RH | 6 mo | 48.53 | Acceptable |
| 40°C/75%RH | 1 mo | 82 | Acceptable for physical parameters |
| 40°C/75%RH | 2 mo | EEE | Difficult to squeeze, Separation FAIL |
| 40°C/75%RH | 3 mo | Above 100 | Hard to Squeeze, Separation |
| 49°C | 4 wk | - | Acceptable for physical parameters |
| 4°C | 24 hr | - | Acceptable for physical parameters |

The results presented in Table 4 (above) show that this formula has very low initial viscosity (around 9 bku) which leads to the composition failing to stand up, and instead flowing off a target surface such as a toothbrush. Upon storage at higher temperatures, the viscosity of the formulation is observed to increase beyond 80 bku.

Based upon the results observed, it is concluded that the formulations containing herbal extracts are not stable in compositions having a silica backbone. Also, it is observed that carboxymethyl cellulose gum system did not produce desired viscosity in both the chalk and silica backbone.

### Comparative Example C

Based upon the observations above, a formulation with Carrageenan Gum system and a water-soluble form of Zinc (Zinc Sulphate) is prepared as shown in Table 5 (below).

**Table 5**

| **Ingredient** | **Wt.%** |
|---|---|
| Humectant (70%) | 17.00 |
| Carrageenan Concentrate | 0.950 |
| Sweetener | 0.150 |
| Purified Water | 30.0 |
| pH Adjusting Agent | 0.50 |
| Maricha (Piper nigrum) | 0.70 |
| Shunthi / Dry Ginger extract (Zingiber officinale) | 0.70 |
| Clove Oil | 0.30 |
| Babool | 0.50 |
| Karpura / Camphor (Cinnamomum Camphora) | 0.50 |
| Thymol | 0.20 |
| Eucalyptus Oil | 0.03 |
| Eugenol | 0.17 |
| Fenugreek | 0.10 |
| Zinc Sulphate | 2.20 |
| Amla Extract | 0.20 |
| Ca Carbonate (RNCC) | 43.0 |
| Anionic Surfactant | 2.0 |
| Menthol | 0.50 |
| Benzyl Alcohol | 0.30 |
| TOTAL | 100.0 |

The composition is evaluated and the results are reported in Table 6 (below).

**Table 6**

| **Condition** | **Time** | **Viscosity (bku)** | **Physical Examination of Dental Cream** |
|---|---|---|---|
| 25C | 0 mo | 16.9 | Stand up, Less viscous appearance, Acceptable |
| 25°C/60%RH | 1 mo | 18.4 | Acceptable for physical parameters |
| 25°C/60%RH | 2 mo | 14.7 | Acceptable |
| 25°C/60%RH | 3 mo | 16.08 | Acceptable |
| 40°C/75%RH | 1 mo | 18.9 | Acceptable for physical parameters |
| 40°C/75%RH | 2 mo | 13.1 | Acceptable |
| 40°C/75%RH | 3 mo | 13.50 | Separation, Acceptable |
| 49°C | 4 wk | - | Discoloration, Acceptable for physical parameters |
| 4°C | 24 hr | - | Stand up, Less viscous Appearance, Acceptable |

The results presented in Table 6 (above) show that this formula has a low initial viscosity (around 17 bku) which can lead to the composition failing to stand up, developed some discoloration and separation upon storage stability testing.

### Comparative Example D

A composition with Carrageenan Gum, Zinc Sulphate, Zinc Oxide and herbal extracts is prepared, as described in Table 7 (below).

**Table 7**

| **Ingredient** | **Wt. %** |
|---|---|
| Humectant (70%) | 17.0 |
| Carrageenan Concentrate | 0.95 |
| Sweetener | 0.15 |
| Purified Water | 28.95 |
| pH Adjusting Agent | 0.50 |
| Maricha (*Piper nigrum*) | 0.70 |
| Shunthi (*Zingiber officinale*) | 0.70 |
| Clove Oil | 0.30 |
| Babool | 0.50 |
| Karpura (*Cinnamomum Camphora*) | 0.50 |
| Thymol | 0.10 |
| Eucalyptus Oil | 0.03 |
| Eugenol | 0.17 |
| Fenugreek | 0.10 |
| Zinc Sulphate | 1.10 |
| Zinc Oxide | 0.50 |
| Amla Extract | 0.20 |
| Thickener | 1.75 |
| Ca Carbonate (RNCC) | 43.0 |
| Anionic Surfactant | 2.0 |
| Menthol | 0.50 |
| Benzyl Alcohol | 0.30 |
| TOTAL | 100.0 |

The composition is evaluated and the results are reported in Table 8 (below).

**Table 8**

| **Condition** | **Time** | **Viscosity (bku)** | **Physical Examination of Dental Cream** |
|---|---|---|---|
| 25°C | 0 mo | 16.9 | Stand up, Less viscous appearance, Acceptable |
| 25°C/60%RH | 1 mo | 21.5 | Acceptable for physical parameters |
| 25°C/60%RH | 2 mo | 19.4 | Acceptable |
| 25°C/60%RH | 3 mo | 21.67 | Acceptable |
| 25°C/60%RH | 6 mo | 24.80 | Pass |
| 40°C/75%RH | 1 mo | 19.5 | Acceptable for physical parameters |
| 40°C/75%RH | 2 mo | 26.3 | Acceptable |
| 40°C/75%RH | 3 mo | 37.74 | Shoulder separation, Acceptable |
| 49°C | 4 wk | - | Discoloration, Acceptable for physical parameters |
| 4°C | 24 hr | - | Acceptable |

The results presented in Table 8 (above) show that this formula has a low initial viscosity (around 17 bku) which can lead to the composition failing to stand up, developed some discoloration and separation upon storage stability testing. After storage at elevated temperature, the formula displayed an increase in viscosity, shoulder separation and discoloration.

### Example 1

A composition containing Carrageenan Gum, Zinc Citrate Trihydrate, Zinc Oxide, Calcium Carbonate base, Herbal Extracts and Essential Oils was prepared, as described in Table 9 (below).

**Table 9**

| **Ingredient** | **Wt.%** |
|---|---|
| Humectant (70%) | 30.00 |
| Carrageenan Concentrate | 0.825 |
| Sweetener | 0.22 |
| Sodium Monofluorophosphate-USP | 0.76 |
| pH Adjusting Agent | 0.50 |
| Purified Water | 18.15 |
| Thickener | 1.75 |
| Refined Natural Calcium Carbonate | 19.00 |
| Precipitated Calcium Carbonate | 21.50 |
| Annionic Surfactant | 2.00 |
| Zinc Oxide | 1.00 |
| Zinc Citrate Trihydrate | 0.50 |
| Desensitizing agent | 0.30 |
| Abrasive | 1.00 |
| Amla Extract | 0.10 |
| Honey Extract | 0.0004 |
| Neem Seed Oil | 0.010 |
| Fennel Oil | 0.0004 |
| Basil Oil | 0.0004 |
| Aloe Vera Extract | 0.0004 |
| Cinnamon Leaf Oil | 0.0004 |
| Flavor Option 1- Herbal 5(containing essential oils) | 1.80 |
| Colorants | 0.28 |
| Benzyl Alcohol | 0.30 |
| Total | 100.0 |

The composition described on Table 9 was evaluated and the results are reported in Table 10 (below).

**Table 10**

| **Condition** | **Time** | **Viscosity (bku)** | **Physical Examination of Dental Cream** |
|---|---|---|---|
| 25°C | 0 mo | 28 | Acceptable |
| 25°C/60%RH | 1 mo | 26.11 | Acceptable |
| 25°C/60%RH | 2 mo | 28 | Acceptable |
| 25°C/60%RH | 3 mo | 25.3 | Acceptable |
| 40°C/75%RH | 1 mo | 23.68 | Acceptable |
| 40°C/75%RH | 2 mo | 24 | Acceptable |
| 40°C/75%RH | 3 mo | 25.9 | Acceptable |

The results described in Table 10 (above) demonstrate that the composition described in Table 9 formula was stable with respect to all the analytical parameters. This chalk base formulation, containing Calcium Carbonate, Carrageenan, Zinc Citrate Trihydrate, Zinc Oxide, and Herbal Extracts and Essential Oil produced a surprisingly stable, efficacious formula having a Natural connotation of taste and color.

### Efficacy Testing

An in vitro bridging study on planktonic bacteria is conducted to compare the antibacterial efficacy of two different flavor compositions, based upon the composition of Example 1, against a Naturals placebo toothpaste without Zinc, and a toothpaste containing Zinc Oxide and Zinc Citrate without Herbs or Essential Oils (Comparative Example E).

The active ingredients in the Naturals Honey & Naturals Cinnamon are shown in Table 11 (below).

**Table 11**

| **Active Antibacterial Ingredient** | **Wt. %** |
|---|---|
| Zinc Oxide | 1 |
| Zinc Citrate | 0.5 |
| Amla Extract | 0.1 |
| Honey Extract | 0.0004 |
| Neem Seed Oil | 0.01 |
| Fennel Oil | 0.0004 |
| Basil Oil | 0.0004 |
| Aloe Vera Extract | 0.0004 |
| Cinnamon Leaf Oil | 0.0004 |
| Flavor (containing essential oils) | 1.8 |

Different essential oils components are used in Naturals Honey & Naturals Cinnamon, in order to produce a different Key signature note:
Example 1 - Naturals Honey: Clove, Camphor, Honey
Example 2 - Naturals Cinnamon: Clove, Camphor, Cinnamon

The Naturals Placebo (not claimed) does not contain Zinc Citrate, Zinc Oxide, Herbal Extracts or Essential oils. The results are reported in Table 12 (below).

**Table 12: Fluorescence values used as measure of % Viability**

| **Samples** | **N** | **Mean** | **Grouping** |
|---|---|---|---|
| Naturals Placebo | 3 | 74.58 | A |
| Ex. 1 - Naturals Honey | 3 | 18.17 | B |
| Ex. 2 - Naturals Cinnamon | 3 | 19.76 | B |
| Comparative Example E | 3 | 18.14 | B |

The data presented in Table 12 (above) show that the compositions of Example 1 and Example 2 significantly outperform a Naturals Placebo in controlling planktonic bacteria (Note that the Group values indicate statistically significant difference between results, i.e. a Group A value shows a high degree of bacterial viability, a Group B value shows a significantly lower level of bacterial viability).

### Example 3: Consumer Perception on the Product

Consumers evaluated compositions prepared using the Naturals Honey flavored composition described above, as for Example 3, which has key flavor notes of Clove, Camphor & Honey and it was found to be authentic and natural by the consumers, also no metallic or Zinc taste was reported by consumers. This flavor was able to mask the high percentage of Zinc in the formula and at the same time produced a consumer perception of Natural flavor notes.

## Claims

1. An oral care composition comprising:
a first source of zinc ions comprising zinc oxide;
a second source of zinc ions comprising zinc citrate;
a gum system comprising carrageenan;
an orally acceptable carrier comprising calcium carbonate; and
a natural ingredient component comprising:
an herbal extract; and
an essential oil.

2. The oral care composition according to claim 1, comprising:
from 0.1 to 5%, by weight, of zinc oxide; and
from 0.05 to 2%, by weight, of zinc citrate.

3. The oral care composition according to claim 1 or 2, comprising:
1 %, by weight of zinc oxide; and
0.5 %, by weight of zinc citrate.

4. The oral care composition according to any foregoing claim, comprising from 0.1 to 5% by weight of herbal extract and, wherein the herbal extract is selected from amla extract, honey extract, almond extract, aloe vera extract, maricha extract, ginger extract, fenugreek, and combinations of two or more thereof.

5. The oral care composition according to any foregoing claim, comprising from 0.1 to 5% by weight of essential oil and, wherein the essential oil is selected from neem seed oil, sesame oil, cinnamon leaf oil, clove oil, thyme oil, eucalyptus oil, , basil oil, eugenol, menthol, babool, camphor, and combinations of two or more thereof.

6. The oral care composition according to any foregoing claim, wherein the herbal extract is selected from amla extract, aloe vera extract, honey extract and a combination thereof; and the essential oil is selected from neem seed oil, basil oil, cinnamon leaf oil, and a combination of two or more thereof.

7. The oral care composition according to any foregoing claim, wherein the gum system comprises carrageenan concentrate.

8. The oral care composition according to any foregoing claim, further comprising a fluoride ion source selected from: stannous fluoride, sodium fluoride, potassium fluoride, sodium monofluorophosphate, sodium fluorosilicate, ammonium fluorosilicate, amine fluoride, ammonium fluoride, and a combination of two or more thereof.

9. The oral care composition according to any foregoing claim, wherein the calcium carbonate is selected from natural calcium carbonate, precipitated calcium carbonate, and combinations thereof.

10. The composition of any preceding claim, for use in treating or preventing a disease or condition of the oral cavity comprising contacting an oral cavity surface of a patient in need thereof with the oral care composition according to any foregoing claim.

11. The composition for use according to claim 10, wherein the disease or condition of the oral cavity is a disease or condition caused by oral bacteria.

12. The oral care composition according to any of claims 1 to 9, wherein the composition comprises clove oil, camphor and honey extract.

## Patentansprüche

1. Mundpflegezusammensetzung, umfassend:
eine erste Quelle von Zinkionen, die Zinkoxid umfasst;
eine zweite Zinkionenquelle, die Zinkcitrat umfasst;
ein Gummisystem das Carrageen umfasst;
einen oral verträglichen Träger, der Calciumcarbonat umfasst; und
eine natürliche Inhaltsstoffkomponente, umfassend:
einen Kräuterextrakt; und
ein ätherisches Öl.

2. Mundpflegezusammensetzung nach Anspruch 1, umfassend:
von 0,1 % bis 5 %, bezogen auf das Gewicht, Zinkoxid; und
0,05 bis 2 %, bezogen auf das Gewicht, Zinkcitrat.

3. Mundpflegezusammensetzung nach Anspruch 1 oder 2, umfassend:
1 %, bezogen auf das Gewicht, Zinkoxid; und
0,5 %, bezogen auf das Gewicht, Zinkcitrat.

4. Mundpflegemittel nach einem beliebigen der vorhergehenden Ansprüche, umfassend 0,1 bis 5 %, bezogen auf das Gewicht, Kräuterextrakt, wobei der Kräuterextrakt ausgewählt ist aus Amla-Extrakt, Honigextrakt, Mandelextrakt, Aloe-Vera-Extrakt, Maricha-Extrakt, Ingwerextrakt, Bockshornklee und Kombinationen von zwei oder mehr davon.

5. Mundpflegemittel nach einem beliebigen der vorhergehenden Ansprüche, umfassend 0,1 bis 5 %, bezogen auf das Gewicht, ätherisches Öl, wobei das ätherische Öl ausgewählt ist aus Neemsamenöl, Sesamöl, Zimtblattöl, Nelkenöl, Thymianöl, Eukalyptusöl, Basilikumöl, Eugenol, Menthol, Babool, Kampfer und Kombinationen von zwei oder mehreren davon.

6. Mundpflegemittel nach einem beliebigen der vorhergehenden Ansprüche, wobei der Kräuterextrakt ausgewählt ist aus Amla-Extrakt, Aloe-Vera-Extrakt, Honigextrakt und einer Kombination davon; und das ätherische Öl ausgewählt ist aus Neemsamenöl, Basilikumöl, Zimtblattöl und einer Kombination von zwei oder mehreren davon.

7. Mundpflegezusammensetzung nach einem der vorstehenden Ansprüche, wobei das Gummisystem Carrageen-Konzentrat umfasst.

8. Mundpflegezusammensetzung nach einem beliebigen der vorhergehenden Ansprüche, weiterhin umfassend eine Fluoridionenquelle, ausgewählt aus: Zinn(II)-fluorid, Natriumfluorid, Kaliumfluorid, Natriummonofluorophosphat, Natriumfluorsilikat, Ammoniumfluorsilikat, Aminfluorid, Ammoniumfluorid und einer Kombination von zwei oder mehreren davon.

9. Mundpflegezusammensetzung nach einem beliebigen der vorhergehenden Ansprüche, wobei das Calciumcarbonat ausgewählt ist aus natürlichem Calciumcarbonat, gefälltem Calciumcarbonat und Kombinationen davon.

10. Zusammensetzung nach einem beliebigen der vorhergehenden Ansprüche zur Verwendung bei der Behandlung oder Vorbeugung einer Krankheit oder eines Zustands der Mundhöhle, umfassend das Inkontaktbringen einer Mundhöhlenoberfläche eines Patienten, der dies benötigt, mit der Mundpflegezusammensetzung nach einem beliebigen der vorhergehenden Ansprüche.

11. Zusammensetzung zur Verwendung nach Anspruch 10, wobei die Krankheit oder der Zustand der Mundhöhle eine Krankheit oder ein Zustand ist, der von oralen Bakterien verursacht wird.

12. Mundpflegezusammensetzung nach einem der Ansprüche 1 bis 9, wobei die Zusammensetzung Nelkenöl, Kampfer und Honigextrakt umfasst.

## Revendications

1. Composition de soins bucco-dentaires comprenant :
une première source d'ions zinc comprenant de l'oxyde de zinc ;
une seconde source d'ions zinc comprenant du citrate de zinc ;
un système de gomme comprenant du carraghénane ;
un excipient oralement acceptable comprenant du carbonate de calcium ; et
un ingrédient naturel comprenant :
un extrait de plante ; et
une huile essentielle.

2. Composition de soins bucco-dentaires selon la revendication 1, comprenant :
de 0,1 à 5 %, en poids, d'oxyde de zinc ; et
de 0,05 à 2 %, en poids, de citrate de zinc.

3. Composition de soins bucco-dentaires selon la revendication 1 ou 2, comprenant :
1 %, en poids d'oxyde de zinc ; et
0,5 %, en poids de citrate de zinc.

4. Composition de soins bucco-dentaires selon l'une quelconque des revendications précédentes, comprenant de 0,1 à 5 % en poids d'extrait végétal et, dans laquelle l'extrait végétal est choisi parmi l'extrait d'amla, l'extrait de miel, l'extrait d'amande, l'extrait d'aloe vera, l'extrait de maricha, l'extrait de gingembre, le fenugrec et les combinaisons de deux ou plus de ceux-ci.

5. Composition de soins bucco-dentaires selon l'une quelconque des revendications précédentes, comprenant de 0,1 à 5 % en poids d'huile essentielle et, dans laquelle l'huile essentielle est choisie parmi l'huile de graines de neem, l'huile de sésame, l'huile essentielle de feuille de cannelier, l'huile essentielle de clou de girofle, l'huile essentielle de thym, l'huile essentielle d'eucalyptus, l'huile essentielle de basilic, l'eugénol, le menthol, le babool, le camphre et les combinaisons de deux ou plus de ceux-ci.

6. Composition de soins bucco-dentaires selon l'une quelconque des revendications précédentes, dans laquelle l'extrait végétal est choisi parmi l'extrait d'amla, l'extrait d'aloe vera, l'extrait de miel et une combinaison de ceux-ci ; et l'huile essentielle est choisie parmi l'huile de graines de neem, l'huile essentielle de basilic, l'huile essentielle de feuille de cannelier et une combinaison de deux ou plus de ceux-ci.

7. Composition de soins bucco-dentaires selon l'une quelconque des revendications précédentes, dans laquelle le système de gomme comprend un concentré de carraghénane.

8. Composition de soins bucco-dentaires selon l'une quelconque des revendications précédentes, comprenant en outre une source d'ions fluorure choisie parmi : le fluorure stanneux, le fluorure de sodium, le fluorure de potassium, le monofluorophosphate de sodium, le fluorosilicate de sodium, le fluorosilicate d'ammonium, le fluorure d'amine, le fluorure d'ammonium et une combinaison de deux ou plus de ceux-ci.

9. Composition de soins bucco-dentaires selon l'une quelconque des revendications précédentes, dans laquelle le carbonate de calcium est choisi parmi le carbonate de calcium naturel, le carbonate de calcium précipité et les combinaisons de ceux-ci.

10. Composition selon l'une quelconque des revendications précédentes, pour une utilisation dans le traitement ou la prévention d'une maladie ou d'une affection de la cavité buccale, comprenant la mise en contact d'une surface de la cavité buccale d'un patient en ayant besoin avec la composition de soins bucco-dentaires selon l'une quelconque des revendications précédentes.

11. Composition pour une utilisation selon la revendication 10, dans laquelle la maladie ou l'affection de la cavité buccale est une maladie ou une affection causée par des bactéries buccales.

12. Composition de soins bucco-dentaires selon l'une quelconque des revendications 1 à 9, dans laquelle la composition comprend de l'huile essentielle de clou de girofle, du camphre et de l'extrait de miel.
